# EUROPEAN PATENT SPECIFICATION

(11) **EP 3 647 785 B1**
(45) Date of publication and mention of the grant of the patent: **20.11.2024**
(21) Application number: 18822661.7
(22) Date of filing: 26.06.2018
(51) Int. Cl.: G01N 33/53, C07K 16/18, G01N 33/543, G01N 33/577

(54) **METHOD FOR MEASURING SERUM AMYLOID A OF VARIOUS ANIMALS AND REAGENT FOR MEASUREMENT THEREOF**
VERFAHREN ZUR MESSUNG VON SERUM-AMYLOID A VERSCHIEDENER TIERE UND REAGENZ ZUR MESSUNG DAVON
PROCÉDÉ DE MESURE DE L'AMYLOÏDE A SÉRIQUE CHEZ DIVERS ANIMAUX ET RÉACTIF DE MESURE UTILISÉ

(30) Priority: 26.06.2017 JP 2017124578
(43) Date of publication of application: 06.05.2020
(73) Proprietor: Eiken Kagaku Kabushiki Kaisha, Taito-ku Tokyo 110-8408 (JP)
(72) Inventor: ASAHI, Yukari, Shimotsuga-gun, Tochigi 329-0114 (JP); WADA, Atsufumi, Shimotsuga-gun, Tochigi 329-0114 (JP)
(74) Representative: Altmann Stößel Dick Patentanwälte PartG mbB
(86) International application number: PCT/JP2018/024114
(87) International publication number: WO 2019/004168

(56) References cited:
- JP-A- H0 843 389
- JP-A- H0 967 398
- US-A1- 2012 322 163
- P. V SYVERSEN ET AL: "The Primary Structure of Serum Amyloid A Protein in the Sheep: Comparison with Serum Amyloid A in Other Species", SCANDINAVIAN JOURNAL OF IMMUNOLOGY, 1 January 1994 (1994-01-01), Oxford, UK, pages 88 - 94, XP055771081, Retrieved from the Internet <URL:https://doi.org/10.1111/j.1365-3083.1994.tb03344.x> DOI: 10.1111/j.1365-3083.1994.tb03344.x
- JACOBSEN ET AL: "Evaluation of a commercially available human serum amyloid A (SAA) turbidometric immunoassay for determination of equine SAA concentrations", VETERINARY JOURNAL, BAILLIERE TINDALL, LONDON, GB, vol. 172, no. 2, 1 September 2006 (2006-09-01), pages 315 - 319, XP005587726, ISSN: 1090-0233, DOI: 10.1016/J.TVJL.2005.04.021
- TAKASHI TAMAMOTO ET AL: "FULL PAPER Clinical Pathology Verification of Measurement of the Feline Serum Amyloid A (SAA) Concentration by Human SAA Turbidimetric Immunoassay and Its Clinical Application", J. VET. MED. SCI, 1 January 2008 (2008-01-01), pages 1247 - 1252, XP055451463, Retrieved from the Internet <URL:https://www.jstage.jst.go.jp/article/jvms/70/11/70_11_1247/_pdf/-char/en>
- CHRISTENSEN, M. ET AL: "Evaluation of an automated assay based on monoclonal anti-human serum amyloid A (SAA) antibodies for measurement of canine, feline and equine SAA", THE VETERINARY JOURNAL, vol. 194, no. 3, 15 June 2012 (2012-06-15), pages 332 - 337, XP055125554, DOI: 10.1016/j.tvjl.2012.05.007
- KJELGAARD-HANSEN, MADS JENS; HANSEN, A. E.; SCHAAP, M. K.: "Determination of feline serum amyloid A (SAA) by means of a commercially available human SAA Turbidimetric immunoassay", ABSTRACTS AND PROCEEDINGS [FOR THE] 5. INTERNATIONAL COLLOQUIUM ON ANIMAL ACUTE PHASE PROTEINS., 2005, pages 51, XP055660457
- SEFERIAN, K. ET AL: "Monoclonal antibodies specific to serum amiloid A from different species", JOURNAL OF VETERINARY INTERNAL MEDICINE (2015 ACVIM FORUM RESEARCH ABTRACT PROGRAM), vol. 29, no. 4, OT10, 27 May 2015 (2015-05-27), pages 1223, XP009518441, ISSN: 0891-6640
- SASAKI, K. ET AL.: "Characterization of monoclonal antibodies specific for feline serum amyloid (SAA) protein", HYBRIDOMA, vol. 20, no. 2, 2001, pages 103 - 108, XP055660503, DOI: 10.1089/02724570152057599
- OHNO, K. ET AL.: "Expression of recombinant feline serum amyloid A (SAA) protein", THE JOURNAL OF VETERINARY MEDICAL SCIENCE, vol. 61, no. 8, August 1999 (1999-08-01), pages 915 - 920, XP055660516, DOI: 10.1292/jvms.61.915

## Description

### [Technical Field]

The present invention relates to a method for measuring serum amyloid A (hereafter, referred to as "SAA") of various animals.

### [Background Art]

SAA is a precursor protein of amyloid A protein that is deposited in tissue upon amyloidosis, which is a serum protein with a molecular weight of approximately 12,000 (Non-Patent Document 1). The serum concentration of SAA is known to increase in inflammatory diseases other than amyloidosis, and SAA is thus recognized as a sensitive inflammation marker (Non-Patent Document 2).

Also, SAA is known to serve as an important inflammation marker in animal species other than humans (Non-Patent Documents 3 to 7).

For example, Patent Document 1 discloses an apparent correlation between the SAA protein level in a milk sample obtained from the breast of a lactating mammal (e.g., bovine) and the inflammatory response level of the breast tissue (i.e., mastitis). Patent Document 2 discloses that infectious diseases can be distinguished from noninfectious causes of diseases based on the SAA concentration in a blood sample obtained from a mammal (e.g., horse) showing abnormal symptoms or actions.

For example, Patent Document 3 discloses a monoclonal antibody that specifically binds to human SAA, and Patent Document 4 discloses a monoclonal antibody that specifically binds to equine SAA. In the past, however, no reagents capable of measuring SAA derived from various animal species including humans involving the use of monoclonal antibodies were known.

### [Prior Art Documents]

### [Patent Documents]

[Patent Document 1] JP 2003-507725 A
[Patent Document 2] WO 2014/118764
[Patent Document 3] JP H09-67398 A (1997)
[Patent Document 4] JP 2008-239511 A

### [Non-Patent Documents]

[Non-Patent Document 1] Husby G. and Natvig J. B., J. Clin. Invest., 1974, Vol. 53, pp. 1054-1061
[Non-Patent Document 2] Yamada, T. et al., Journal of Clinical Laboratory Medicine, 1988, 32:2, pp. 167-172
[Non-Patent Document 3] Nunokawa Y. et al., J. Vet. Med. Sci., 1993, 55 (6), pp. 1011-1016
[Non-Patent Document 4] Kajikawa, T. et al., J. Vet. Med. Sci., 1996, 58 (11), pp. 1141-1143
[Non-Patent Document 5] Tamamoto, T. et al., J. Vet. Med. Sci., 2008, 70 (11), pp. 1247-1252
[Non-Patent Document 6] Petersen, H. H. et al., Vet. Res., 2004, 35(2), pp. 163-187
[Non-Patent Document 7] Tamamoto, T., Journal of Hokkaido Veterinary Medical Association, 2014, 58, pp. 539-543

### [Summary of the Invention]

### [Objects to Be Attained by the Invention]

Under the above circumstances, it is an object of the present invention to provide a reagent that can measure SAA derived from various animal species.

### [Means for Attaining the Objects]

The present inventors have conducted concentrated studies in order to attain the above object. As a result, they found an animal species cross-reactive monoclonal antibody that recognizes SAA derived from various animal species from among anti-human SAA monoclonal antibodies. This has led to the completion of the present invention.

Specifically, the present invention includes the following.
(1) A method for immunologically measuring serum amyloid A comprising a step of measuring serum amyloid A using a reagent for measuring serum amyloid A from a plurality of animal species, said reagent comprising an animal species cross-reactive anti-serum amyloid A monoclonal antibody or a fragment thereof,
   wherein said animal species cross-reactive anti-serum amyloid A monoclonal antibody or a fragment thereof specifically binds to, as the serum amyloid A protein epitope, the amino acid residues in the 80th to the 90th positions from the N terminus of the mature human serum amyloid A1 protein consisting of the amino acid sequence as shown in SEQ ID NO: 1,
   wherein said fragment of monoclonal antibody is selected from the group consisting of Fab, Fab', F(ab')₂, Fv, Fd and Fabc,

   wherein the plurality of animal species are a plurality of animal species selected from the group consisting of a human, a bovine, a dog, a cat, a rabbit, a horse, a monkey, a pig, a sheep, a donkey, and a mouse, and
   wherein the method for immunological measurement is the latex turbidimetric immunoassay.

Japanese Patent Application No. 2017-124578 is the priority document of the present application.

### [Effects of the Invention]

With the method for immunologically measuring SAA according to the present invention, SAA, which is an inflammation marker for various animal species, can be easily detected or measured.

### [Brief Description of the Drawings]

Fig. 1 shows the results of measuring a bovine specimen using a latex reagent composed of polystyrene latex particles of 130 nm in diameter and an anti-human SAA polyclonal antibody immobilized thereon.
Fig. 2 shows the results of screening of an anti-human SAA monoclonal antibody using a bovine specimen.
Fig. 3 shows the results of measuring a dilution series of serum specimens with known human SAA concentration using a latex reagent composed of polystyrene latex particles of 130 nm in diameter and the monoclonal antibody (Clone 15), which was confirmed to be reactive with bovine SAA, immobilized thereon.

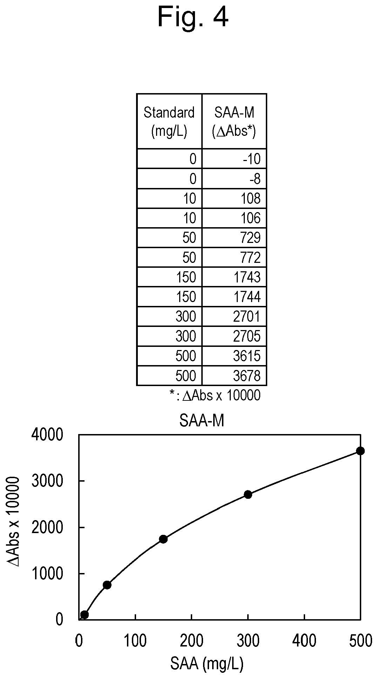

Fig. 4 shows the results of measuring a dilution series of serum specimens with known human SAA concentration (calibration curve) using a latex reagent (SAA-M) prepared by immobilizing the animal species cross-reactive anti-SAA monoclonal antibody (Clone 15) that binds to SAA by recognizing, as an epitope, a region in the vicinity of an amino acid residue in the 90th position from the N terminus of the mature human SAA1 protein consisting of the amino acid sequence as shown in SEQ ID NO: 1 or a region in the vicinity of a corresponding amino acid residue in other mature SAA protein to two types of polystyrene latex particles (i.e., particles of 220 nm in diameter and particles of 80 nm in diameter) and mixing the two types of polystyrene latex particles at 2:1.
Fig. 5 shows the results of measuring the bovine, dog, and cat serum specimens using a latex reagent (SAA-M) prepared by immobilizing the animal species cross-reactive anti-SAA monoclonal antibody (Clone 15) to two types of latex particles (i.e., particles of 220 nm in diameter and particles of 80 nm in diameter) and mixing the two types of latex particles at 2:1.
Fig. 6 shows the results of measuring SAA in serum samples obtained from various animals (a bovine, a horse, a monkey, a donkey, a sheep, a pig, a capybara, a mouse, and a rabbit) using a latex reagent prepared by immobilizing the animal species cross-reactive anti-SAA monoclonal antibody (Clone 15) to polystyrene latex particles of 215 nm in diameter.
Fig. 7 shows the results of examination of reactivity of the anti-human SAA monoclonal antibodies (Clones 15, 18, and 21) with serum specimens obtained from various animals (a human, a cat, a dog, a horse, a bovine, a monkey, a capybara, and a mouse).
Fig. 8 shows a map of arrays used for epitope mapping of the anti-human SAA monoclonal antibodies (Clones 15, 18, and 21).
Fig. 9 shows the results of examination of reactivity of the anti-human SAA monoclonal antibodies (Clones 15, 18, and 21) with each peptide derived from the mature human SAA1 protein.
Fig. 10 shows the results of examination of reactivity of the anti-human SAA monoclonal antibodies (Clones 15, 18, and 21) with each peptide derived from the mature human SAA1 protein: (A) shows fluorescent images on arrays used for epitope mapping; (B) shows a map of arrays used for epitope mapping; and (C): shows a comparison of amino acid sequences of each peptide derived from the mature human SAA1 protein.

### [Embodiments of the Invention]

Hereafter, the present invention is described in detail.

The reagent for measuring SAA obtained from a plurality of (i.e., 2 or more) animal species used in the method according to the present invention (hereafter, referred to as "the reagent for measuring SAA according to the present disclosure") comprises an animal species cross-reactive anti-SAA monoclonal antibody or a fragment thereof. The animal species cross-reactive anti-SAA monoclonal antibody or a fragment thereof used in the method of the present invention (hereafter, referred to as "the monoclonal antibody or a fragment thereof according to the present disclosure") is a monoclonal antibody capable of recognizing SAA derived from various animal species and binding to such protein or a fragment thereof. With the use of the method for measuring SAA according to the present invention, SAA as an inflammation marker in various animal species can be immunologically measured.

Examples of SAA that the monoclonal antibody or a fragment thereof used in the method according to the present invention can recognize include SAA derived from animal species, such as a human, a bovine, a dog, a cat, a rabbit, a horse, a monkey, a pig, a sheep, a donkey, and a mouse.

The monoclonal antibody or a fragment thereof used in the method according to the present invention preferably binds to SAA by recognizing, as an epitope, the amino acid residues in the 80th to the 90th positions from the N terminus of the mature human serum amyloid A1 protein consisting of the amino acid sequence as shown in SEQ ID NO: 1. The monoclonal antibody or a fragment thereof used in the method according to the present invention that binds to such epitope exhibits high binding property to SAA obtained from a plurality of animal species. A region of amino acid residues in other mature SAA protein (e.g., a mature SAA protein derived from an animal species other than a human) corresponding to a region in the vicinity of an amino acid residue in the 90th position from the N terminus of the mature human SAA1 protein consisting of the amino acid sequence as shown in SEQ ID NO: 1 to which the monoclonal antibody or a fragment thereof used in the method according to the present invention binds as an epitope, can be determined via, for example, alignment comparison between the amino acid sequence of the mature human SAA1 protein and the amino acid sequence of other mature SAA protein in accordance with a conventionally known method.

The monoclonal antibody used in the method according to the present invention may be of any immunoglobulin (Ig) class (e.g., IgA, IgG, IgE, IgD, IgM, or IgY) and any subclass (e.g., IgG1, IgG2, IgG3, IgG4, IgA1, or IgA2). The immunoglobulin light chain may be a κ chain or λ chain.

Examples of the monoclonal antibody fragment used in the method according to the present invention include Fab, Fab', F(ab')₂, Fv, Fd, and Fabc. Methods for preparing such fragments are known in the art. For example, such fragments can be obtained by digestion of antibody molecules with a protease such as papain or pepsin or via a known genetic engineering technique.

The monoclonal antibody used in the method according to the present invention can be obtained by preparing hybridomas from antibody-producing cells obtained from nonhuman mammals immunized with antigens (human SAA) (e.g., spleen cells or lymphatic cells) and myeloma cells via fusion, proliferating the obtained hybridomas in a selection medium containing hypoxanthine, aminopterin, and thymidine ("HAT medium"), cloning cells producing monoclonal antibodies exhibiting specific affinity to the antigens used for immunization (human SAA) or fragment peptide antigens thereof (e.g., a peptide antigen comprising a region in the vicinity of an amino acid residue in the 90th position from the N terminus of the mature human SAA1 protein consisting of the amino acid sequence as shown in SEQ ID NO: 1), obtaining anti-human SAA monoclonal antibody-producing cell lines, proliferating the anti-human monoclonal antibody-producing cells in the mouse abdominal cavity, and purifying the antibody from the resulting ascites fluid. Examples of nonhuman mammals include rodents such as mice and rats. Myeloma cells derived from the same animal as the immunized animals are preferably used, and examples thereof include mouse myeloma cells and rat myeloma cells. Antibody-producing cells can be fused to myeloma cells with the use of polyethylene glycol (PEG) or via electrical fusion.

The prepared monoclonal antibody can be purified by a method known in the art, such as chromatography using a protein A or a protein G column, ion exchange chromatography, hydrophobic chromatography, salting out with ammonium sulfate, gel filtration, or affinity chromatography, in adequate combination.

The present invention relates to a method for immunologically measuring SAA comprising a step of measuring SAA using the reagent for measuring SAA as disclosed herein. Specifically, SAA in a biological sample derived from various animal species is brought into contact with the monoclonal antibody or a fragment thereof as disclosed herein in the reagent for measuring SAA as disclosed herein to cause an antigen-antibody reaction, and SAA in the sample is detected or measured based on the formed immune complex.

A biological sample that comprises or may comprise SAA is sufficient, and examples thereof include whole blood, serum, plasma, urine, puncture fluid, sweat, saliva, lymph, and spinal fluid samples.

Examples of methods for immunological measurement include: single radial immunodiffusion (not part of the invention) comprising observing the expression of a precipitate line formed by an immune complex resulting from binding of the monoclonal antibody or a fragment thereof as disclosed herein to SAA in the biological sample on an agar plate; enzyme immunoassay (EIA) (not part of the invention) or radioimmunoassay (not part of the invention) involving the use of the monoclonal antibody or a fragment thereof as disclosed herein labeled with an enzyme or radioactive isotope; and use of an insoluble carrier comprising the monoclonal antibody or a fragment thereof as disclosed herein immobilized thereon. Examples of insoluble carriers include particles such as latex particles (such as polyethylene or polystyrene particles), alumina particles (not encompassed by the invention), silica particles (not encompassed by the invention), gold colloid particles (not encompassed by the invention), and magnetic particles (not encompassed by the invention). Among such insoluble carriers, latex particles are preferable, and polystyrene latex particles are particularly preferable.

Insoluble carrier particles are preferably of 50 to 500 nm in diameter, and more preferably of 75 to 350 nm in diameter.

An antibody or a fragment thereof can be immobilized on an insoluble carrier in accordance with a conventional technique. Specifically, an antibody or a fragment thereof is mixed with an insoluble carrier, and an antibody or a fragment thereof is allowed to physically adsorb to the insoluble carrier surface. Thus, an antibody or a fragment thereof can be immobilized on the insoluble carrier.

When an insoluble carrier comprising an amino or carboxyl group introduced on its surface is used, an antibody or a fragment thereof can be immobilized on the insoluble carrier surface via a chemical bond involving the use of a glutaraldehyde or carbodiimide reagent.

Examples of methods involving the use of insoluble carriers include: a latex turbidimetric immunoassay involving the use of latex particles comprising the monoclonal antibody or a fragment thereof used in the method according to the present invention immobilized thereon; gold colloid-based agglutination colorimetry (not part of the invention) involving the use of gold colloid particles comprising the monoclonal antibody or a fragment thereof as disclosed herein immobilized on gold colloids; and immunochromatographic assays (not part of the invention) involving the use of the monoclonal antibody or a fragment thereof as disclosed herein labeled with metal colloids and a capture antibody that captures an immune complex of the monoclonal antibody or a fragment thereof as disclosed herein and SAA on a membrane such as nitrocellulose membrane. According to the latex turbidimetric immunoassay, specifically, latex particles comprising the monoclonal antibody or a fragment thereof used in the method according to the present invention immobilized on the latex are allowed to react with SAA in a biological sample, and SAA is measured based on agglutination of latex particles as a result of formation of the immune complex and changes in turbidity caused by latex agglutination. According to immunochromatographic assays (not part of the invention), a biological sample is supplied onto a membrane such as nitrocellulose membrane, SAA in the biological sample reacts with the monoclonal antibody or a fragment thereof as disclosed herein in a label-reagent-retaining region that retains the monoclonal antibody or a fragment thereof as disclosed herein labeled with metal colloids or the like to form an immune complex, the immune complex migrates on the membrane by the capillary action, the immune complex is captured by a capture antibody immobilized in a given position on the membrane, and SAA is detected based on the color developed as a result of the capturing.

As described above, the reagent for measuring SAA as disclosed herein can be used for a method for immunologically measuring SAA. When the method for immunological measurement involves the use of an insoluble carrier, for example, the reagent for measuring SAA as disclosed herein can contain an insoluble carrier comprising the monoclonal antibody or a fragment thereof as disclosed herein immobilized thereon, such as a latex solution containing latex particles. When immunochromatographic assays (not part of the invention) are performed, the reagent for measuring SAA as disclosed herein can be used for an immunochromatographic device composed of an insoluble carrier comprising the monoclonal antibody or a fragment thereof as disclosed herein immobilized thereon, such as gold colloids, and a membrane such as nitrocellulose membrane comprising the monoclonal antibody or a fragment thereof as disclosed herein immobilized thereon (e.g., a membrane such as nitrocellulose membrane supported on a carrier comprising a sample supply region, a label-reagent-retaining region that retains the monoclonal antibody or a fragment thereof as disclosed herein labeled with metal colloids or the like, and a detection region comprising a capture antibody immobilized on a given position).

In addition to the monoclonal antibody or a fragment thereof used in the method according to the present invention, the reagent for measuring SAA used in the method according to the present invention can comprise a buffer that imparts a pH level necessary for immunological reaction, a reaction enhancer that accelerates immunological reaction, a reaction stabilizer or blocker that suppresses non-specific reaction, a preservative that improves storage stability of a reagent, such as sodium azide, and the like.

Examples of buffers include the following.

### Good's buffers:

(2-(N-morpholino)ethanesulfonic acid; abbreviated as "MES";
piperazine-N,N'-bis(2-ethanesulfonic acid); abbreviated as "PIPES";
N-(2-acetamido)-2-aminoethanesulfonic acid; abbreviated as "ACES";
N,N-bis(2-hydroxyethyl)-2-aminoehtanesulfonic acid; abbreviated as "BES";
bis(2-hydroxyethyl)iminotris(hydroxymethyl)methane; abbreviated as "Bis-Tris";
3-[N,N-bis(2-hydroxyethyl)amino]-2-hydroxypropanesulfonic acid; abbreviated as "DIPSO";
N-2-hydroxyethylpiperazine-N'-3-propanesulfonic acid; abbreviated as "EPPS";
N-2-hydroxyethylpiperazine-N'-2-ethanesulfonic acid; abbreviated as "HEPES";
N-2-hydroxyethylpiperazine-N'-2-hydroxypropane-3-sulfonic acid; abbreviated as "HEPPSO";
3-(N-morpholino)propanesulfonic acid; abbreviated as "MOPS";
3-(N-morpholino)-2-hydroxypropanesulfonic acid; abbreviated as "MOPSO";
pioerazine-N,N'-bis(2-hydroxypropanesulfonic acid); abbreviated as "POPSO";
N-tris(hydroxymethyl)methyl-3-aminopropanesulfonic acid; abbreviated as "TAPS";
N-tris(hydroxymethyl)methyl-2-hydroxy-3-aminopropanesulfonic acid; abbreviated as "TAPSO"; and
N-tris(hydroxymethyl)methyl-2-aminoethanesulfonic acid; abbreviated as "TES".

### Other buffers:

2-amino-2-hydroxymethyl-1,3-propanediol; also referred to as "tris(hydroxymethyl)aminomethane";
Phosphate buffers;
ammonium buffer.

Among such buffers, Good's buffers, such as HEPES and PIPES, adjust a pH level to an advantageous level for the immunological reaction. In addition, the influence thereof imposed on proteins is small. Thus, such buffers are particularly preferable. A pH level necessary for the immunological reaction is 5 to 11, and it is preferably 6 to 9.

As a reaction enhancer, for example, polyethylene glycol and dextran sulfate are known. In addition, BSA (bovine serum albumin), animal serum, IgG, IgG fragments (Fab and Fc), albumin, milk protein, amino acid, polyamino acid, choline, a polysaccharide such as sucrose, gelatin, degraded gelatin, casein, a polyhydric alcohol such as glycerin, and the like are known to effectively stabilize the reaction or inhibit the non-specific reaction in the immunological reaction in the form of reaction stabilizers or blockers.

The reagent for measuring SAA used in the method according to the present invention comprising various components described above can be supplied in a liquid or dry state. In order to realize distribution of the reagent in a liquid state, the reagent may further be supplemented with, for example, various surfactants, saccharides, or inactive proteins, so as to improve protein stability. Such stabilizers are also effective as stabilizers or excipients when drying the reagent.

### [Examples]

Hereafter, the present invention is described in greater detail with reference to the Examples.

In the Comparative Examples and the Examples below, epitopes of the mature human SAA1 protein (the amino acid sequence as shown in SEQ ID NO: 1) to which monoclonal antibodies bind are as described below:
Clone 17: a region in the vicinity of the amino acid residue in the 30th position from the N terminus;
Clone 14: a region in the vicinity of the amino acid residue in the 15th position from the N terminus;
Clones 15 and 18: a region in the vicinity of the amino acid residue in the 90th position from the N terminus; and
Clones 21 and 25: unknown, although an epitope for Clone 21 is deduced to be the same as that for Clone 15 etc. based on reactivity with bovine SAA.

### [Comparative Example 1] Measurement of bovine specimen using latex reagent comprising anti-human SAA polyclonal antibody immobilized thereon

### 1. Objectives

Whether or not bovine SAA could be measured using a latex reagent comprising the anti-human SAA polyclonal antibody immobilized on polystyrene latex particles was examined.

### (1) Immobilization of anti-human SAA polyclonal antibody on polystyrene latex particles

The anti-human SAA polyclonal antibody was immobilized on polystyrene latex particles of 130 nm in diameter.

The anti-human SAA polyclonal antibody was immobilized on the polystyrene latex particles in accordance with a conventional technique. Specifically, the anti-human SAA polyclonal antibody was mixed with polystyrene latex, so as to allow the anti-human SAA polyclonal antibody to physically adsorb on the polystyrene latex surface. Thus, the anti-human SAA polyclonal antibody was immobilized on the polystyrene latex particles.

### 2. Materials and Methods

Bovine plasma specimens sampled with time before and after surgery were measured by using the above latex reagent with the Hitachi 7170S automatic analyzer (Hitachi High-Technologies). Specifically, 2.0 µl of the plasma specimen was mixed with 100 µl of the first reagent (50 mM HEPES buffer, pH 7.4), the mixture was incubated at 37°C for 5 minutes, 100 µl of the second reagent (a polyclonal antibody-immobilized latex solution containing 10 mM HEPES buffer, pH 7.4) was added thereto, the reaction was allowed to proceed at 37°C, and changes in the absorbance were measured at the dual wavelength of 800/570 nm (sub wavelength/main wavelength) for approximately 5 minutes after the second reagent was added.

The SAA concentration in the plasma specimens was calculated from the calibration curve obtained by measuring the sample with known SAA concentration.

### 3. Results

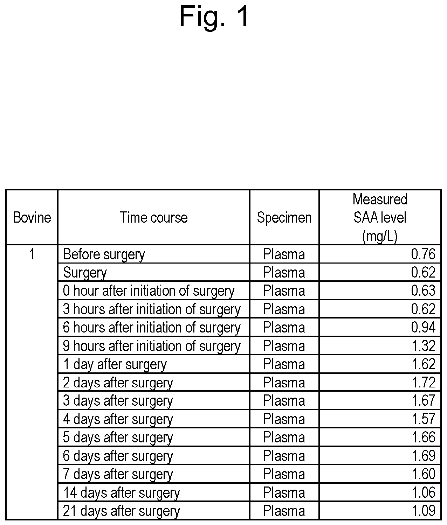

The SAA concentration in the bovine plasma specimens sampled with time before and after surgery was 0.62 to 1.72 mg/l, which was below the measurement range of the SAA reagent prepared with the polyclonal antibody (5 to 500 mg/l) (Fig. 1).

### 4. Conclusions

The SAA reagent prepared with the use of the anti-human SAA polyclonal antibody exhibited low reactivity with bovine SAA, and, accordingly, bovine SAA could not be measured. While bovine SAA could be measured via EIA involving the use of the polyclonal antibody as described in Example 1, it could not be measured via the latex turbidimetric immunoassay.

### [Example 1] Screening of monoclonal antibody using bovine specimen

### 1. Objective

The monoclonal antibody reacting with bovine SAA was screened.

### 2. Materials and Methods

The bovine plasma specimens sampled before and after surgery were immobilized on a microplate (antigen immobilization), the anti-human SAA polyclonal antibodies and the anti-human SAA monoclonal antibodies (Clones 14, 15, 17, 18, 21, and 25 shown in Table 1 of Patent Document 3) were allowed to react therewith, and reactivity was then examined via EIA in which the POD-labeled anti-rabbit IgG antibody or the POD-labeled anti-rat IgG antibody was allowed to react.

### 3. Results

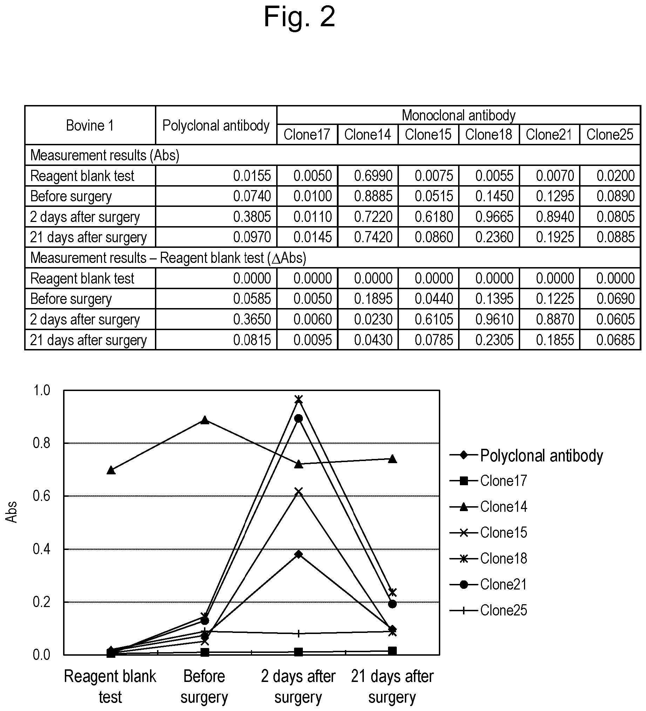

Concerning reactivity with the bovine plasma specimens at 2 days after surgery, the reactivity of Clone 18 was the highest, followed by Clone 21, Clone 15, and the polyclonal antibody in descending order, and no difference was observed in reactivity of other clones before and after surgery (Fig. 2).

### 4. Conclusions

It was confirmed that the anti-human SAA polyclonal antibody reacted with the bovine plasma while the reaction level was low and the monoclonal antibodies (Clone 17 and Clone 25) did not react with the bovine plasma specimens at the acute stage. Among the monoclonal antibodies used for screening, 3 monoclonal antibody clones (Clone 18, Clone 21, and Clone 15) that would react with the bovine plasma specimens at the acute stage were identified.

### [Example 2] Examination for preparation of the reagent for measuring animal SAA ("SAA-M") and measurement of specimens

### 1. Objective

The latex reagents for measuring animal SAA were examined.

### 2. Materials and Methods

Latex reagents were prepared with the use of the anti-human SAA monoclonal antibody (Clone 15) and polystyrene latex particles of 130 nm in diameter or of 220 nm and 80 nm in diameter, and the dilution series of serum specimens with known SAA concentration and bovine, dog, and cat serum specimens were measured. As a comparative example, the measurement with commercially available LZ test "Eiken" SAA (Eiken Chemical Corporation) was also carried out.

The anti-human SAA monoclonal antibody was immobilized on the polystyrene latex particles in accordance with a known method. Specifically, the anti-human SAA monoclonal antibody was mixed with polystyrene latex, so as to allow the anti-human SAA monoclonal antibody to physically adsorb on the polystyrene latex surface. Thus, the anti-human SAA monoclonal antibody was immobilized on the polystyrene latex particles.

The dilution series of serum specimens with known SAA concentration (human SAA) and bovine, dog, and cat serum specimens were measured with the use of the Hitachi 7170S automatic analyzer (Hitachi High-Technologies). Specifically, 2.0 µl of the serum specimen was mixed with 100 µl of the first reagent (50 mM HEPES buffer, pH 7.4), the mixture was incubated at 37°C for 5 minutes, 100 µl of the second reagent (a monoclonal antibody-immobilized latex solution containing 10 mM HEPES buffer, pH 7.4) was added thereto, the reaction was allowed to proceed at 37°C, and changes in the absorbance were measured at the dual wavelength of 800/570 nm (sub wavelength/main wavelength) for approximately 5 minutes after the second reagent was added.

The SAA concentration in the bovine, dog, and cat serum specimens were calculated from the calibration curve obtained by measuring the sample with known SAA concentration.

### 3. Results

### 3-1. Reagent preparation with monoclonal antibody

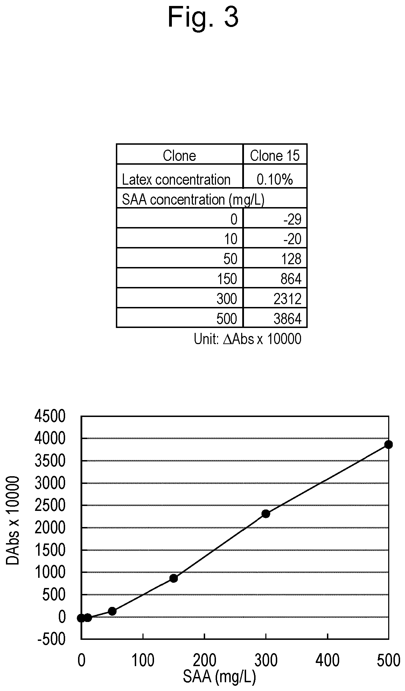

Fig. 3 shows the results of measurement of the dilution series of serum specimens with known SAA concentration (human SAA) with the use of the SAA reagent comprising polystyrene latex particles of 130 nm in diameter and the monoclonal antibody (Clone 15) immobilized thereon. With the use of the SAA reagent comprising Clone 15 immobilized thereon, a fluctuation was observed in changes in the absorbance (ΔAbs) in the dilution series, and the calibration curve was thus obtained.

The monoclonal antibody (Clone 15) was immobilized on two types of latex particles (i.e., particles of 220 nm in diameter and particles of 80 nm in diameter), and a latex reagent (SAA-M) comprising these two 2 types of latex particles at 2:1 was prepared. Fig. 4 shows the results of measurement of the dilution series of serum specimens with known SAA concentration (calibration curve).

### 3-2. Measurement of animal specimens

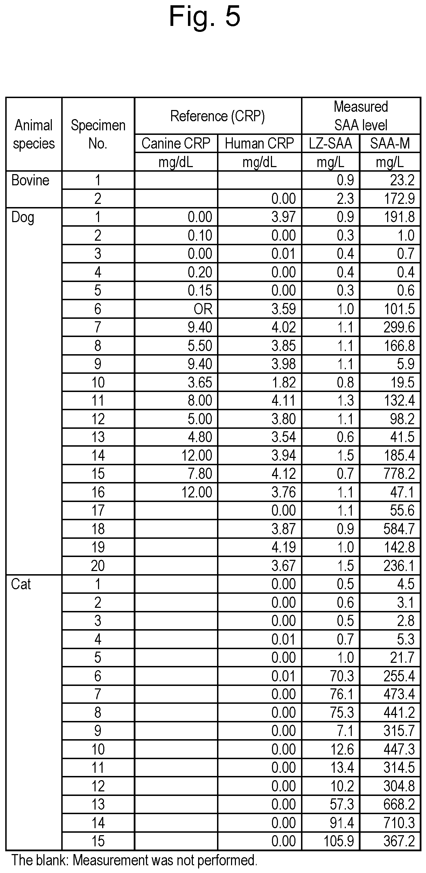

Fig. 5 shows the results of measurement of the bovine, dog, and cat serum specimens. SAA in the bovine, dog, and cat specimens could be measured with the use of the latex reagent (SAA-M) prepared by immobilizing the monoclonal antibody (Clone 15) on two types of latex particles (i.e., particles of 220 nm in diameter and particles of 80 nm in diameter). With the use of the LZ test "Eiken" SAA (LZ-SAA), in contrast, the SAA level of the bovine and dog serum specimens was below the measurement range of the LZ test "Eiken" SAA; that is, the SAA level in the bovine and dog specimens could not be measured with the use of the LZ test "Eiken" SAA. In the case of the dog specimen, a larger fluctuation was observed compared with the level measured with the use of a commercially available reagent for measuring canine CRP, and a larger fluctuation was observed in the cat specimen compared with the results attained with the use of the LZ test "Eiken" SAA.

### 4. Conclusions

With the use of the monoclonal antibody (Clone 15) binding to SAA by recognizing, as an epitope, a region in the vicinity of an amino acid residue in the 90th position from the N terminus of the mature human SAA1 protein consisting of the amino acid sequence as shown in SEQ ID NO: 1, SAA latex reagents reacting with SAA obtained from a bovine, a dog, and a cat can be prepared.

### [Example 3] Measurement of SAA in various animal specimens

### 1. Objective

Whether or not SAA of various animal species could be measured with the use of the monoclonal antibody (Clone 15) binding to SAA by recognizing, as an epitope, a region in the vicinity of an amino acid residue in the 90th position from the N terminus of the mature human SAA1 protein consisting of the amino acid sequence as shown in SEQ ID NO: 1 was examined.

### 2. Materials and Methods

SAA in various animal species was measured using the latex reagent immobilizing the monoclonal antibody (Clone 15) (animal SAA reagent).

As a comparative example, measurement was carried out in the same manner with the use of the LZ test "Eiken" SAA (LZ-SAA) described in Example 2. An animal species (rabbit) that would not react with the LZ test "Eiken" SAA (LZ-SAA) was subjected to measurement of CRP that would be elevated in case of inflammatory diseases as with SAA for reference.

The animal SAA reagent was prepared in the same manner as in Example 2 except for the use of polystyrene latex particles of 215 nm in diameter.

SAA in various animal specimens was measured with the use of the Hitachi 7170S automatic analyzer (Hitachi High-Technologies). Specifically, 3.0 µl each of various specimens was mixed with 100 µl of the first reagent (50 mM HEPES buffer, pH 7.4), the mixture was incubated at 37°C for 5 minutes, 100 µl of the second reagent (a monoclonal antibody-immobilized latex solution containing 10 mM HEPES buffer, pH 7.4) was added thereto, the reaction was allowed to proceed at 37°C, and changes in the absorbance were measured at wavelength of 600 nm for approximately 5 minutes after the second reagent was added.

The SAA concentration in various animal specimens was calculated from the calibration curve obtained by measuring the sample with known SAA concentration.

### 3. Results

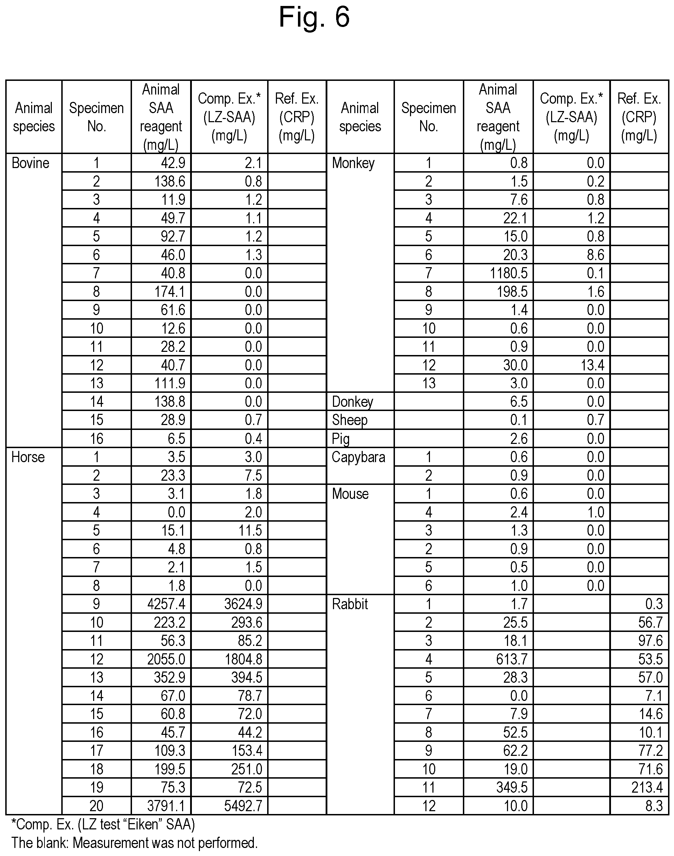

Fig. 6 shows the results of measurement.

While SAA could not be measured in the comparative example (the LZ test "Eiken" SAA) (below the measurement sensitivity), SAA in specimens obtained from various animals (i.e., a bovine, a horse, a monkey, a donkey, a sheep, a pig, a mouse, and a rabbit) could be measured with the use of the animal SAA reagent (the monoclonal antibody (Clone 15) binding to SAA by recognizing, as an epitope, a region in the vicinity of an amino acid residue in the 90th position from the N terminus of the mature human SAA1 protein consisting of the amino acid sequence as shown in SEQ ID NO: 1; corresponding to "the reagent for measuring SAA according to the present disclosure").

### 4. Conclusions

With the use of the animal SAA reagent (the monoclonal antibody (Clone 15)), SAA in various animal species can be measured.

### [Example 4] Evaluation of SAA reagent using antibodies obtained from various clones

### 1. Objective

The monoclonal antibodies obtained from Clone 18 and Clone 21 binding to SAA by recognizing, as an epitope, a region in the vicinity of an amino acid residue in the 90th position from the N terminus of the mature human SAA1 protein consisting of the amino acid sequence as shown in SEQ ID NO: 1 were examined as to whether or not SAA in various animal specimens could be measured as with the case of the monoclonal antibody of Clone 15.

### 2. Materials and Methods

Various animal serum samples obtained from a human, a cat, a dog, a horse, a bovine, a monkey, a capybara, and a mouse were immobilized on a microplate (antigen immobilization), the anti-SAA monoclonal antibodies obtained from Clone 15, Clone 18, and Clone 21 were allowed to react therewith, and reactivity was then examined via EIA in which the POD-labeled anti-rat IgG antibody was allowed to react.

### 3. Results

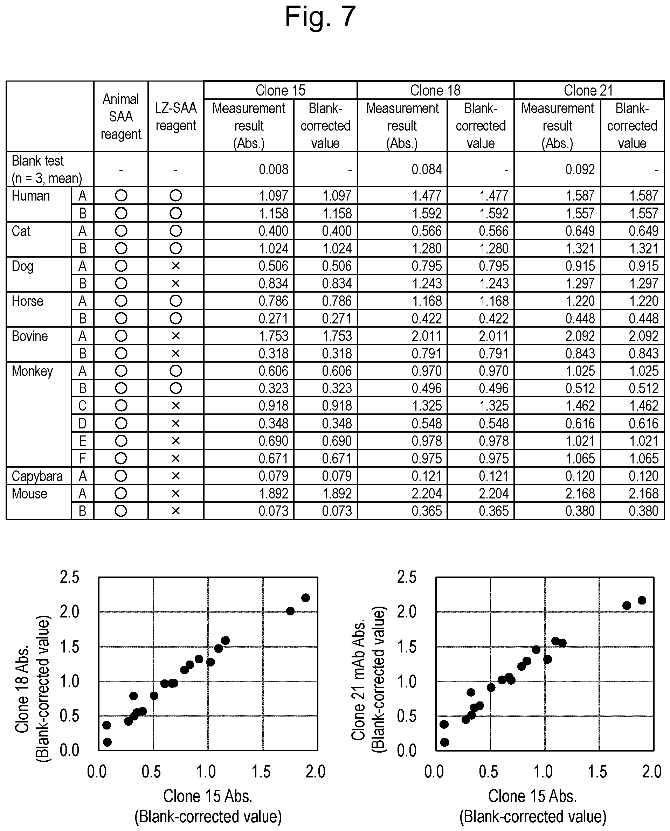

The results of measurement are shown in Fig. 7. The symbols "∘" and "×" used in the table shown in Fig. 7 indicate the presence and the absence of the reaction (presence: ∘, absence: ×), respectively. Concerning the "Blank-corrected value" shown in the table in Fig. 7, the reaction is determined to have occurred (+) when the blank-corrected value is 0.020 or higher by taking dispersion into consideration.

With the use of "the monoclonal antibody of Clone 18 that binds to SAA by recognizing, as an epitope, a region in the vicinity of an amino acid residue in the 90th position from the N terminus of the mature human SAA1 protein consisting of the amino acid sequence as shown in SEQ ID NO: 1 according to the present disclosure" and "the monoclonal antibody of Clone 21 that binds to SAA by recognizing, as an epitope, a region in the vicinity of an amino acid residue in the 90th position from the N terminus of the mature human SAA1 protein consisting of the amino acid sequence as shown in SEQ ID NO: 1 according to the present disclosure," SAA in various animal specimens could be measured as with the use of "the monoclonal antibody of Clone 15 that binds to SAA by recognizing, as an epitope, a region in the vicinity of an amino acid residue in the 90th position from the N terminus of the mature human SAA1 protein consisting of the amino acid sequence as shown in SEQ ID NO: 1 according to the present disclosure." As shown in the chart shown in Fig. 7, a correlational coefficient of Clone 18 to Clone 15 was 0.982, and that of Clone 21 to Clone 15 was 0.975.

### 4. Conclusions

It was suggested that the monoclonal antibody of Clone 18 and the monoclonal antibody of Clone 21 exhibit equivalent properties as the monoclonal antibody of Clone 15.

### [Example 5] Epitope mapping of antibodies obtained from various clones

### 1. Objective

The amino acid sequences of the mature human SAA1 protein consisting of the amino acid sequence as shown in SEQ ID NO: 1 to which the monoclonal antibodies obtained from Clone 15, Clone 18, and Clone 21 would bind as epitopes were examined.

### 2. Materials and Methods

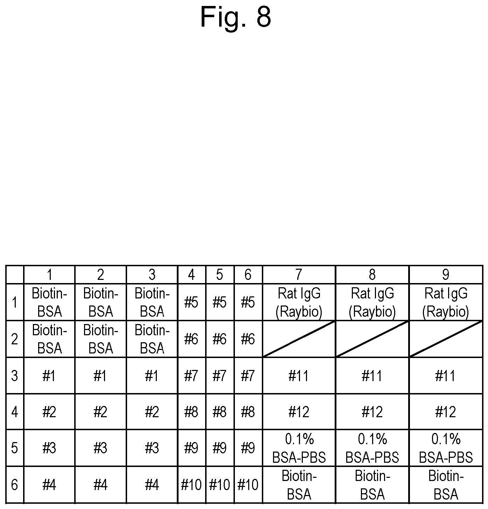

As shown in Table 1 and Fig. 8, peptides derived from the mature human SAA1 protein were immobilized on glass slides (antigen immobilization) to prepare arrays.

Subsequently, the anti-SAA monoclonal antibodies obtained from Clone 15, Clone 18, and Clone 21 were allowed to react with the peptides on the arrays, and the biotin-labeled goat anti-rat IgG antibody was then allowed to react therewith. Thereafter, fluorescence-labeled streptavidin was allowed to react with the arrays, and reactivity of anti-SAA monoclonal antibodies to the peptides was examined.

**[Table 1]**

| No. | Sequence | Positions in SEQ ID NO: 1 | SEQ ID NO: |
|---|---|---|---|
| Peptide #1 | HGAEDSLADQAANEWGRSGKDPNHF | 71 to 95 | 2 |
| Peptide #2 | HGAEDSLADQAANEW | 71 to 85 | 3 |
| Peptide #3 | GAEDSLADQAANEWG | 72 to 86 | 4 |
| Peptide #4 | AEDSLADQAANEWGR | 73 to 87 | 5 |
| Peptide #5 | EDSLADQAANEWGRS | 74 to 88 | 6 |
| Peptide #6 | DSLADQAANEWGRSG | 75 to 89 | 7 |
| Peptide #7 | SLADQAANEWGRSGK | 76 to 90 | 8 |
| Peptide #8 | LADQAANEWGRSGKD | 77 to 91 | 9 |
| Peptide #9 | ADQAANEWGRSGKDP | 78 to 92 | 10 |
| Peptide #10 | DQAANEWGRSGKDPN | 79 to 93 | 11 |
| Peptide #11 | QAANEWGRSGKDPNH | 80 to 94 | 12 |
| Peptide #12 | AANEWGRSGKDPNHF | 81 to 95 | 13 |

| | | | |
|---|---|---|---|
| Positive Controls Biotin-BSA Rat IgG (Raybiotech) Negative Controls 0.1% BSA-PBS | | | |

Positive controls shown in Table 1 and Fig. 8 are as shown below:
Biotin-BSA: biotin-labeled BSA was immobilized on glass slides instead of a peptide derived from a mature human SAA1 protein; and
Rat IgG (Raybiotech): biotin-labeled goat anti-rat IgG antibody was immobilized on glass slides instead of a peptide derived from a mature human SAA1 protein.

### 3. Results

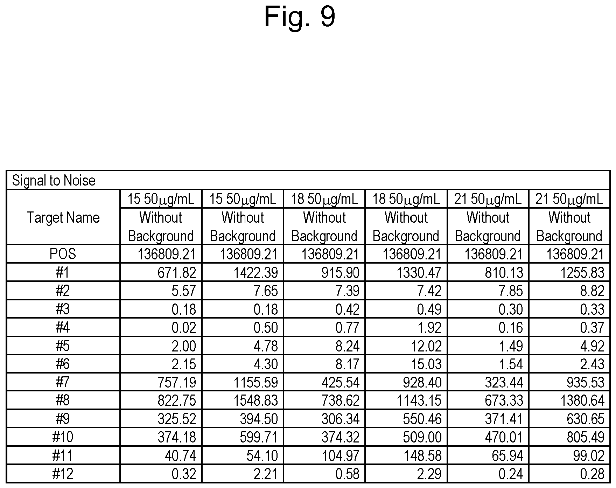

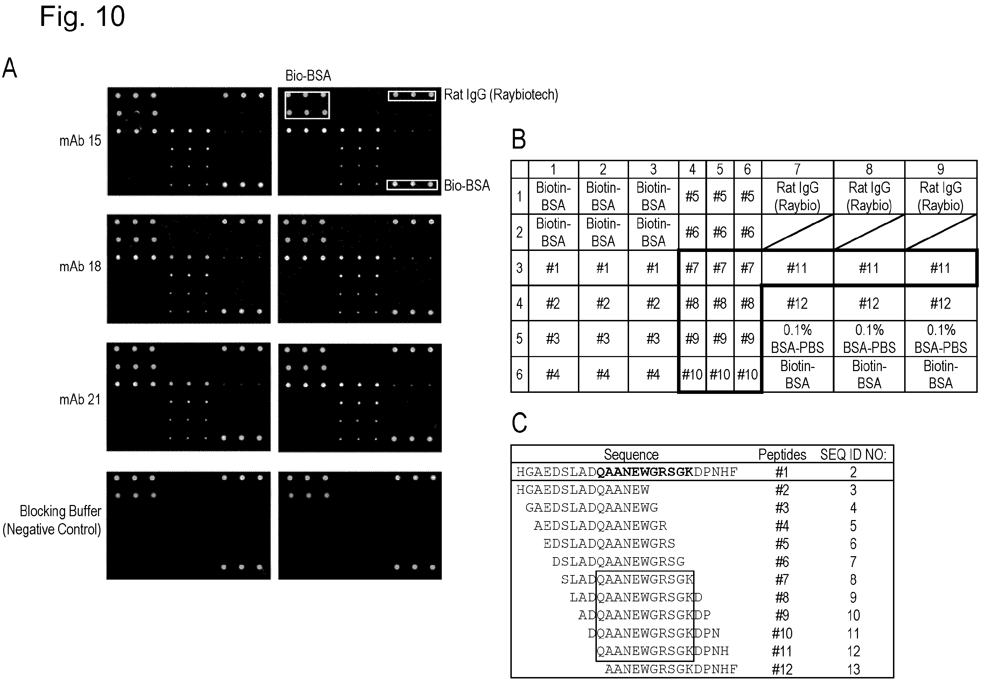

The results are shown in Figs. 9 and 10.

As shown in Figs. 9 and 10, the anti-SAA monoclonal antibodies obtained from Clone 15, Clone 18, and Clone 21 were found to sufficiently bind to Peptides #7 to #11.

### 4. Conclusions

The anti-SAA monoclonal antibodies obtained from Clone 15, Clone 18, and Clone 21 sufficiently bound to Peptides #7 to #11. Accordingly, it was suggested that such antibodies bind to the mature human SAA1 protein by recognizing, as an epitope, the amino acid sequence common among Peptides #7 to #11 (QAANEWGRSGK: SEQ ID NO: 14) (Fig. 10C). Such epitope corresponds to an amino acid sequence in the 80th to the 90th positions from the N terminus of the amino acid sequence as shown in SEQ ID NO: 1.

## Claims

1. A method for immunologically measuring serum amyloid A comprising a step of measuring serum amyloid A using a reagent for measuring serum amyloid A from a plurality of animal species, said reagent comprising an animal species cross-reactive anti-serum amyloid A monoclonal antibody or a fragment thereof,
wherein said animal species cross-reactive anti-serum amyloid A monoclonal antibody or a fragment thereof specifically binds to, as the serum amyloid A protein epitope, the amino acid residues in the 80th to the 90th positions from the N terminus of the mature human serum amyloid A1 protein consisting of the amino acid sequence as shown in SEQ ID NO: 1,
wherein said fragment of monoclonal antibody is selected from the group consisting of Fab, Fab', F(ab')₂, Fv, Fd and Fabc,
wherein the plurality of animal species are a plurality of animal species selected from the group consisting of a human, a bovine, a dog, a cat, a rabbit, a horse, a monkey, a pig, a sheep, a donkey, and a mouse, and
wherein the method for immunological measurement is the latex turbidimetric immunoassay.

## Patentansprüche

1. Verfahren zur immunologischen Messung von Serum-Amyloid A, umfassend einen Schritt, bei dem Serum-Amyloid A unter Verwendung eines Reagenz zum Messen von Serum-Amyloid A aus mehreren Tierspezies gemessen wird, wobei das Reagenz einen für Tierspezies kreuzreaktiven monoklonalen Anti-Serum-Amyloid-A-Antikörper oder ein Fragment davon umfasst,
wobei der für Tierspezies kreuzreaktive monoklonale Anti-Serum-Amyloid-A-Antikörper oder ein Fragment davon an, als das Serum-Amyloid-A-Protein-Epitop, die Aminosäurereste in Position 80 bis 90 vom N-Terminus des reifen Humanserum-Amyloid-A1-Proteins, das aus der Aminosäuresequenz gemäß SEQ ID NO: 1 besteht, spezifisch bindet,
wobei das Fragment von monoklonalem Antikörper aus der Gruppe bestehend aus Fab, Fab', F(ab')₂, Fv, Fd and Fabc ausgewählt ist,
wobei es sich bei den mehreren Tierspezies um mehrere Tierspezies handelt, die aus der Gruppe bestehend aus Mensch, Rind, Hund, Katze, Kaninchen, Pferd, Affe, Schwein, Schaf, Esel und Maus ausgewählt sind, und
wobei es sich bei der immunologischen Messung um den turbidimetrischen Latex-Immunoassay handelt.

## Revendications

1. Procédé pour la mesure de manière immunologique de l'amyloïde A sérique comprenant une étape de mesure de l'amyloïde A sérique en utilisant un réactif pour la mesure de l'amyloïde A sérique à partir d'une pluralité d'espèces animales, ledit réactif comprenant un anticorps monoclonal d'amyloïde A anti-sérique à réactivité croisée pour des espèces animales ou un fragment correspondant,
ledit anticorps monoclonal d'amyloïde A anti-sérique à réactivité croisée pour des espèces animales ou ledit fragment correspondant se liant spécifiquement aux, en tant que l'épitope de protéine d'amyloïde A sérique, résidus d'acides aminés dans les positions 80^{e} aux positions 90^{e} depuis la terminaison N de la protéine amyloïde A1 sérique humaine mature constituée de la séquence d'acides aminés telle qu'indiquée dans la SEQ ID NO:1,
ledit fragment d'anticorps monoclonal étant choisi dans le groupe constitué par Fab, Fab', F(ab')₂, Fv, Fd et Fabc,
la pluralité d'espèces animales étant une pluralité d'espèces animales choisies dans le groupe constitué par un humain, un bovin, un chien, un chat, un lapin, un cheval, un singe, un cochon, un mouton, un âne et une souris, et
le procédé pour la mesure immunologique étant le dosage immunologique turbidimétrique au latex.
